# EUROPEAN PATENT APPLICATION

(11) **EP 2 940 096 A1**
(43) Date of publication of application: **04.11.2015**
(21) Application number: 13866796.9
(22) Date of filing: 13.06.2013
(51) Int. Cl.: C09K 11/06, H01L 51/50

(54) **COMPOUND FOR ORGANIC OPTOELECTRONIC ELEMENT, ORGANIC LIGHT-EMITTING ELEMENT COMPRISING SAME, AND DISPLAY DEVICE COMPRISING THE ORGANIC LIGHT-EMITTING ELEMENT**

(30) Priority: 31.12.2012 KR 20120158170
(71) Applicant: Cheil Industries Inc., Gumi-si, Gyeongsangbuk-do 730-030 (KR)
(72) Inventor: KIM, Hyun-Jung, Uiwang-si Gyeonggi-do 437-711 (KR); SHIN, Chang-Ju, Uiwang-si Gyeonggi-do 437-711 (KR); JEONG, Soo-Young, Uiwang-si Gyeonggi-do 437-711 (KR); KANG, Dong-Min, Uiwang-si Gyeonggi-do 437-711 (KR); KANG, Eui-Su, Uiwang-si Gyeonggi-do 437-711 (KR); WON, Jong-Woo, Uiwang-si Gyeonggi-do 437-711 (KR); LEE, Nam-Heon, Uiwang-si Gyeonggi-do 437-711 (KR); LEE, Hyon-Gyu, Uiwang-si Gyeonggi-do 437-711 (KR); JEONG, Woo-Seok, Uiwang-si Gyeonggi-do 437-711 (KR); JEONG, Ho-Kuk, Uiwang-si Gyeonggi-do 437-711 (KR); CHAE, Mi-Young, Uiwang-si Gyeonggi-do 437-711 (KR)
(74) Representative: Michalski Hüttermann & Partner Patentanwälte mbB
(86) International application number: PCT/KR2013/005238
(87) International publication number: WO 2014/104499

(57) **Abstract**

A compound for an organic optoelectronic device, an organic light-emitting device including the same, and a display device including the organic light-emitting device are disclosed, and the organic light-emitting device has improved life-span characteristics due to excellent electrochemical and thermal stability and high luminous efficiency at a low driving voltage by a compound for an organic optoelectronic device represented by Chemical Formula 1.

## Description

### [Technical Field]

A compound for an organic optoelectronic device having excellent life-span, efficiency, electrochemical stability, and thermal stability, an organic light-emitting device including the compound, and a display device including the organic light-emitting device are disclosed.

### [Background Art]

An organic optoelectronic device is a device requiring a charge exchange between an electrode and an organic material by using holes or electrons.

An organic optoelectronic device may be classified as follows in accordance with its driving principles. A first organic optoelectronic device is an electronic device driven as follows: excitons are generated in an organic material layer by photons from an external light source; the excitons are separated into electrons and holes; and the electrons and holes are transferred to different electrodes as a current source (voltage source).

A second organic optoelectronic device is an electronic device driven as follows: a voltage or a current is applied to at least two electrodes to inject holes and/or electrons into an organic material semiconductor positioned at an interface of the electrodes, and the device is driven by the injected electrons and holes.

Examples of an organic optoelectronic device include an organic photoelectric device, an organic light-emitting device, an organic solar cell, an organic photo conductor drum, an organic transistor, and the like, which require a hole injecting or transport material, an electron injecting or transport material, or a light emitting material.

Particularly, an organic light-emitting device (OLED) has recently drawn attention due to an increase in demand for flat panel displays. In general, organic light emission refers to conversion of electrical energy into photo-energy.

Such an organic light-emitting device converts electrical energy into light by applying current to an organic light emitting material. It has a structure in which a functional organic material layer is interposed between an anode and a cathode. The organic material layer includes a multi-layer including different materials, for example a hole injection layer (HIL), a hole transport layer (HTL), an emission layer, an electron transport layer (ETL), and an electron injection layer (EIL), in order to improve efficiency and stability of an organic light-emitting device.

In such an organic light-emitting device, when a voltage is applied between an anode and a cathode, holes from the anode and electrons from the cathode are injected to an organic material layer and recombined to generate excitons having high energy. The generated excitons generate light having certain wavelengths while shifting to a ground state.

Recently, it has become known that a phosphorescent light emitting material may be used for a light emitting material of an organic light-emitting device in addition to the fluorescent light emitting material. Such a phosphorescent material emits lights by transporting the electrons from a ground state to an exited state, non-radiance transiting of a singlet exciton to a triplet exciton through intersystem crossing, and transiting a triplet exciton to a ground state to emit light.

As described above, in an organic light-emitting device, an organic material layer includes a light emitting material and a charge transport material, for example a hole injection material, a hole transport material, an electron transport material, an electron injection material, and the like.

The light emitting material is classified as blue, green, and red light emitting materials according to emitted colors, and yellow and orange light emitting materials to emit colors approaching natural colors.

When one material is used as a light emitting material, a maximum light emitting wavelength is shifted to a long wavelength or color purity decreases because of interactions between molecules, or device efficiency decreases because of a light emitting quenching effect. Therefore, a host/dopant system is included as a light emitting material in order to improve color purity and increase luminous efficiency and stability through energy transfer.

In order to implement excellent performance of an organic light-emitting device, a material constituting an organic material layer, for example a hole injection material, a hole transport material, a light emitting material, an electron transport material, an electron injection material, and a light emitting material such as a host and/or a dopant, should be stable and have good efficiency. However, development of an organic material layer forming material for an organic light-emitting device has thus far not been satisfactory and thus there is a need for a novel material. This material development is also required for other organic optoelectronic devices.

The low molecular organic light-emitting device is manufactured as a thin film in a vacuum deposition method and can have good efficiency and life-span performance. A polymer organic light-emitting device is manufactured in an inkjet or spin coating method has an advantage of low initial cost and being large-sized.

Both low molecular organic light emitting and polymer organic light-emitting devices have an advantage of self-light emitting, high speed response, wide viewing angle, ultra-thin, high image quality, durability, large driving temperature range, and the like. In particular, they have good visibility due to self-light emitting characteristics compared with a conventional LCD (liquid crystal display) and have an advantage of decreasing thickness and weight of LCD up to a third, because they do not need a backlight.

In addition, since they have a response speed 1000 time faster microsecond unit than LCD, they can realize a perfect motion picture without after-image. Based on these advantages, they have been remarkably developed to have 80 times efficiency and more than 100 times life-span since they come out for the first time in the late 1980s. Recently, they keep being rapidly larger such as a 40-inch organic light-emitting device panel.

They are simultaneously required to have improved luminous efficiency and life-span in order to be larger. Herein, their luminous efficiency need smooth combination between holes and electrons in an emission layer. However, since an organic material in general has slower electron mobility than hole mobility, it has a drawback of inefficient combination between holes and electrons. Accordingly, while increasing electron injection and mobility from a cathode and simultaneously preventing movement of holes is required.

Accordingly, there has been a strong need for an organic compound having excellent electron injection and mobility, and high electrochemical stability.

### [DISCLOSURE]

### [Technical Problem]

A compound for an organic optoelectronic device that may act as hole injection and transport or electron injection and transport material, and also act as a light emitting host along with an appropriate dopant is provided.

An organic light emitting device having excellent life-span, efficiency, driving voltage, electrochemical stability and thermal stability and a display device including the same are provided.

### [Technical Solution]

In one embodiment of the present invention, a compound for an organic optoelectronic device represented by the following Chemical Formula 1 is provided.

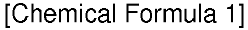

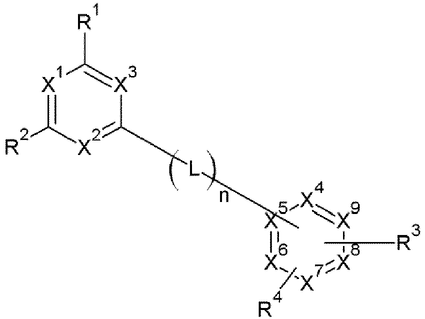

In Chemical Formula 1, X¹ to X⁹ are each independently CR' or N, at least two of the X¹ to X³ is N, at least one of the X⁴ to X⁹ is N, R¹ to R⁴ and R' are each independently hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 alkylthiol group, a substituted or unsubstituted C6 to C20 arylthiol group, a substituted or unsubstituted C1 to C20 heterocyclothiol group, a substituted or unsubstituted C1 to C20 ureide group, a substituted or unsubstituted C3 to C40 silyl group, or a combination thereof, R³ and R⁴ may be linked to each other to form a fused ring, L is a substituted or unsubstituted C2 to C30 heteroarylene group, and n is an integer ranging from 1 to 3.

In another embodiment of the present invention, an organic light-emitting device includes an anode, a cathode, and at least one or more organic thin layer between the anode and the cathode, wherein at least one of the organic thin layers includes the compound for an organic optoelectronic device.

In yet another embodiment of the present invention, a display device including the organic light-emitting device is provided.

### [Advantageous Effects]

A compound having high hole or electron transport properties, film stability thermal stability and high triplet exciton energy is provided.

Such a compound may be used as a hole injection/transport material, host material, or an electron injection/transport material of an emission layer. The organic optoelectronic device using the same has improved life-span characteristics due to excellent electrochemical and thermal stability, and high luminous efficiency at a low driving voltage.

### [Description of the Drawings]

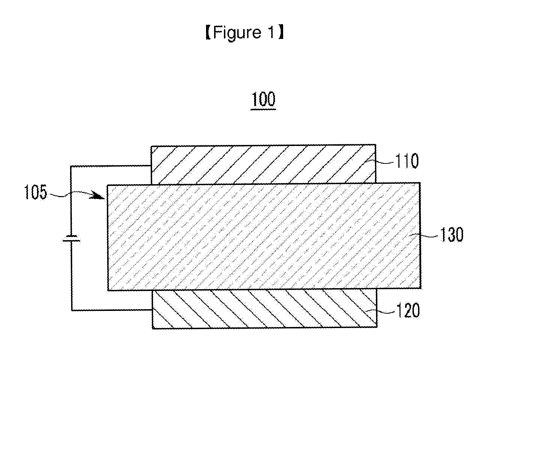

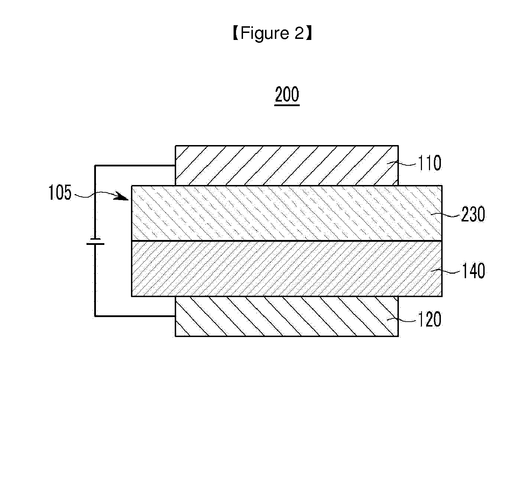

FIGS. 1 and 2 are cross-sectional views showing organic light-emitting devices according to various embodiments of the present invention using a compound for an organic optoelectronic device according to one embodiment of the present invention.

### [Mode for Invention]

Hereinafter, embodiments of the present invention are described in detail. However, these embodiments are exemplary, and do not limit the present invention, and the present invention is defined by the scope of the claims which will be described later.

In the present specification, when specific definition is not otherwise provided, "substituted" refers to one substituted with deuterium, a halogen, hydroxy group, an amino group, a substituted or unsubstituted C1 to C30 amine group, a nitro group, a substituted or unsubstituted C3 to C40 silyl group, a C1 to C30 alkyl group, a C1 to C10 alkylsilyl group, a C3 to C30 cycloalkyl group, a C6 to C30 aryl group, a C1 to C20 alkoxy group, a fluoro group, a C1 to C10 trifluoroalkyl group such as trifluoromethyl group and the like, or a cyano group, instead of at least one hydrogen of a substitutent or a compound.

Two substitutents of the substituted halogen, hydroxy group, amino group, substituted or unsubstituted C1 to C20 amine group, nitro group, substituted or unsubstituted C3 to C40 silyl group, C1 to C30 alkyl group, C1 to C10 alkylsilyl group, C3 to C30 cycloalkyl group, C6 to C30 aryl group, C1 to C20 alkoxy group, fluoro group, C1 to C10 trifluoroalkyl group such as trifluoromethyl group and the like or cyano group may be fused with each other to form a ring.

In the present specification, when specific definition is not otherwise provided, "hetero" refers to one including 1 to 3 hetero atoms selected from the group consisting of N, O, S, and P, and remaining carbons in one compound or substituent.

In the present specification, when a definition is not otherwise provided, "alkyl group" refers to an aliphatic hydrocarbon group. The alkyl group may be "a saturated alkyl group" without a double bond or a triple bond.

The alkyl group may be a branched, linear, or cyclic alkyl group.

The "alkenyl group" refers to a substituent consisting of at least one carbon-carbon double bond of at least two carbons, and the "alkynylene group" refers to a substituent consisting of at least one carbon-carbon triple bond of at least two carbons.

The alkyl group may be a C1 to C20 alkyl group. More specifically, the alkyl group may be a C1 to C10 alkyl group or a C1 to C6 alkyl group.

For example, a C1 to C4 alkyl group may have 1 to 4 carbon atoms and may be selected from the group consisting of methyl, ethyl, propyl, iso-propyl, n-butyl, iso-butyl, sec-butyl, and t-butyl.

Specific examples of the alkyl group may be a methyl group, an ethyl group, a propyl group, an isopropyl group, a butyl group, an isobutyl group, a t-butyl group, a pentyl group, a hexyl group, a cyclopropyl group, a cyclobutyl group, a cyclopentyl group, a cyclohexyl group, and the like.

"Aromatic group" refers to a cyclic functional group where all elements have p-orbitals, and these p-orbitals forms conjugation. Specific examples are aryl group and a heteroaryl group.

"Aryl group" refers to a monocyclic or fused ring polycyclic (i.e., rings sharing adjacent pairs of carbon atoms) functional group.

"Heteroaryl group" refers to an aryl group including 1 to 3 hetero atoms selected from the group consisting of N, O, S, P, and Si and remaining carbons. The heteroaryl group may be a fused ring where each ring may include the 1 to 3 heteroatoms.

In the present specification, hole characteristics refer to characteristics that holes formed in the anode is easily injected into the emission layer and transported in the emission layer due to conductive characteristics according to HOMO level.

Electron characteristics refer to characteristics that electron formed in the cathode is easily injected into the emission layer and transported in the emission layer due to conductive characteristics according to LUMO level.

A compound for an organic optoelectronic device according to one embodiment of the present invention may have a structure of including various substituents in a core that at least three heteroaryl groups are consecutively linked.

The core structure may be used as a light emitting material, a hole injection material or a hole transport material of an organic optoelectronic device. Particularly, it may be suitable as a hole injection material or a hole transport material.

The compound for an organic optoelectronic device includes a core part and various substituents for a substitutent for substituting the core part and thus may have various energy bandgaps.

The compound may have an appropriate energy level depending on the substituents and thus, may fortify hole transport capability or electron transport capability of an organic optoelectronic device and bring about excellent effects on efficiency and driving voltage and also, have excellent electrochemical and thermal stability and thus, improve life-span characteristics during the operation of the organic optoelectronic device.

According to one embodiment of the present invention, the compound for an organic optoelectronic device may be represented by the following Chemical Formula 1.

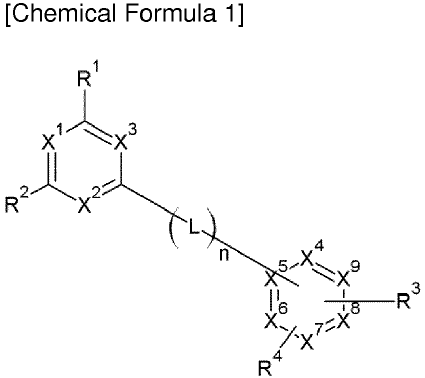

In Chemical Formula 1, X¹ to X⁹ are each independently CR' or N, at least two of the X¹ to X³ is N, at least one of the X⁴ to X⁹ is N, R¹ to R⁴ and R' are each independently hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 alkylthiol group, a substituted or unsubstituted C6 to C20 arylthiol group, a substituted or unsubstituted C1 to C20 heterocyclothiol group, a substituted or unsubstituted C1 to C20 ureide group, a substituted or unsubstituted C3 to C40 silyl group, or a combination thereof, R³ and R⁴ may be linked to each other to form a fused ring, L is a substituted or unsubstituted C2 to C30 heteroarylene group, and n is an integer ranging from 1 to 3.

The compound including the core structure including the three heteroaryl groups linked to each other has an appropriate energy level due to its substituents and may fortify electron transport capability of an organic optoelectronic device. When the compound is applied to an organic optoelectronic device, efficiency and a driving voltage of the device may be improved. The compound may improve life-span characteristics of an organic optoelectronic device due to improved electrochemical and thermal stability.

More specifically, the R¹ to R⁴ are each independently a substituted or unsubstituted phenyl group, a substituted or unsubstituted naphthyl group, a substituted or unsubstituted anthracenyl group, a substituted or unsubstituted phenanthryl group, a substituted or unsubstituted naphthacenyl group, a substituted or unsubstituted pyrenyl group, a substituted or unsubstituted biphenylyl group, a substituted or unsubstituted p-terphenyl group, a substituted or unsubstituted m-terphenyl group, a substituted or unsubstituted chrysenyl group, a substituted or unsubstituted triphenylenyl group, a substituted or unsubstituted perylenyl group, a substituted or unsubstituted indenyl group, a substituted or unsubstituted furanyl group, a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, a substituted or unsubstituted phenoxazinyl group, or a combination thereof, but are not limited thereto.

The R¹ and R² may be each independently a substituted or unsubstituted C6 to C30 aryl group. More specifically, the R¹ and R² may be a fused substituted or unsubstituted C6 to C30 aryl group.

Specific examples of the R¹ and R² may be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted pyrenyl group, a substituted or unsubstituted fluorenyl group, a substituted or unsubstituted p-terphenyl group, a substituted or unsubstituted m-terphenyl group, a substituted or unsubstituted perylenyl group.

The L may be a substituted or unsubstituted C2 to C30 heteroarylene group including one or two nitrogen. The L may make electrons be transported smoothly. That is, it may function as a functional group withdrawing electrons and thus make transport rates of electrons to be similar to those of holes.

In addition, the compound may have increased thermal stability due to a bulk aryl group. More specifically, the substituents may be appropriately selected or modified depending on required characteristics of a device.

A conjugation length all over the compound is determined by selectively adjusting the L, and thus, triplet energy bandgaps may be controlled. Thereby, characteristics of materials required in organic photoelectric device may be realized. In addition, the triplet energy bandgaps may be controlled by changing ortho, para, or meta bonding positions.

Specific examples of the L may be a substituted or unsubstituted imidazolyl group, a substituted or unsubstituted carbazolyl group, a substituted or unsubstituted benzimidazolyl group, a substituted or unsubstituted pyridinyl group, a substituted or unsubstituted pyrimidinyl group, a substituted or unsubstituted triazinyl group, a substituted or unsubstituted pyrazinyl group, a substituted or unsubstituted pyridazinyl group, a substituted or unsubstituted quinolinyl group, a substituted or unsubstituted isoquinolinyl group, a substituted or unsubstituted naphpyridinyl group, a substituted or unsubstituted quinoxalinyl group, a substituted or unsubstituted quinazolinyl group, a substituted or unsubstituted acridinyl group, a substituted or unsubstituted phenanthrolinyl group, or a combination thereof.

More specifically, the compound for an organic optoelectronic device may be represented by the following Chemical Formula 2.

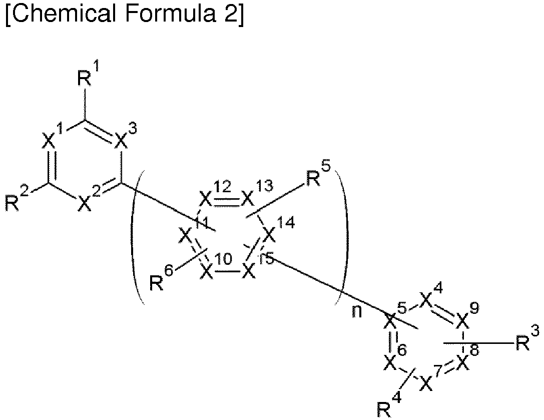

In the above Chemical Formula 2, X¹ to X¹⁵ are each independently CR' or N, at least two of the X¹ to X³ are N, at least one of the X⁴ to X⁹ is N, at least one of the X¹⁰ to X¹⁵ is N, R¹ to R⁶ and R' are each independently hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 alkylthiol group, a substituted or unsubstituted C6 to C20 arylthiol group, a substituted or unsubstituted C1 to C20 heterocyclothiol group, a substituted or unsubstituted C1 to C20 ureide group, a substituted or unsubstituted C3 to C40 silyl group, or a combination thereof, R³ and R⁴ may be linked to each other to form a fused ring, R⁵ and R⁶ may be linked to each other to form a fused ring, and n is an integer ranging from 1 to 3.

At least one of the X¹⁰ to X¹⁵ may be N.

The n may be 1, and in a compound for an organic optoelectronic device according to one embodiment of the present invention, R¹ and R² may be each independently hydrogen, deuterium, a naphthyl group, a phenanthrenyl group, or an anthracenyl group, and R⁴, R⁷ and R⁸ are hydrogen, but is not limited thereto.

More specifically, the compound for an organic optoelectronic device may be represented by the following Chemical Formula 3.

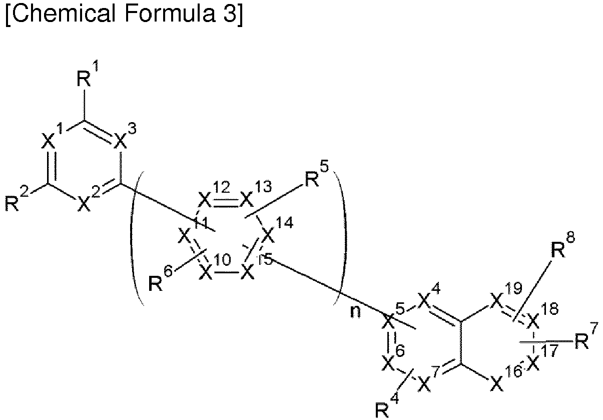

In Chemical Formula 3, X¹ to X⁷, X¹⁰ to X¹⁵ and X¹⁶ to X¹⁹ are each independently CR' or N, at least two of the X¹ to X³ are N, at least one of the X⁴ to X⁷ and X¹⁶ to X¹⁹ is N, at least one of the X¹⁰ to X¹⁵ is N, R¹ R⁴ to R⁸ and R' are each independently hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 alkylthiol group, a substituted or unsubstituted C6 to C20 arylthiol group, a substituted or unsubstituted C1 to C20 heterocyclothiol group, a substituted or unsubstituted C1 to C20 ureide group, a substituted or unsubstituted C3 to C40 silyl group, or a combination thereof, R⁷ and R⁸ may be linked to each other to form a fused ring, R⁵ and R⁶ may be linked to each other to form a fused ring, and n is an integer ranging from 1 to 3.

In the case of the structure of the Chemical Formula 3 where the fused heteroaryl group is linked to substituents, electron transport capability of the compound may be fortified. Efficiency and a driving voltage of an device using the same may be improved. The compound may improve life-span characteristics of an organic optoelectronic device due to improved electrochemical and thermal stability.

In the above Chemical Formula 3, at least one of the X⁴ to X⁷ may be N and the X¹⁶ to X¹⁹ may be CR'. Or, the X⁴ to X⁷ may be CR', and at least one of the X¹⁶ to X¹⁹ may be N. In addition, in the above Chemical Formula 3, R¹ and R² may be each independently hydrogen, deuterium, a naphthyl group, a phenanthrenyl group, or an anthracenyl group, and the R⁴, R⁷ and R⁸ may be hydrogen, but are not limited thereto.

More specifically, the compound for an organic optoelectronic device may be represented by the following Chemical Formula 4.

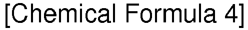

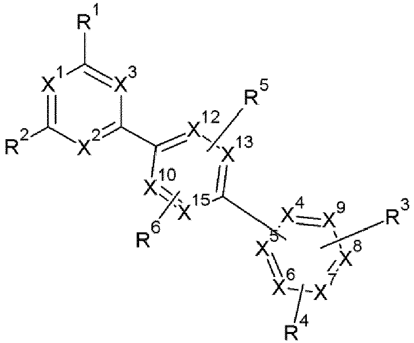

In Chemical Formula 4, X¹ to X⁹, X¹⁰, X¹², X¹³ and X¹⁵ are each independently CR' or N, at least two of the X¹ to X³ are N, at least one of the X⁴ to X⁹ is N, at least one of the X¹⁰, X¹², X¹³ and X¹⁵ is N, R¹ to R⁶ and R' are each independently hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 alkylthiol group, a substituted or unsubstituted C6 to C20 arylthiol group, a substituted or unsubstituted C1 to C20 heterocyclothiol group, a substituted or unsubstituted C1 to C20 ureide group, a substituted or unsubstituted C3 to C40 silyl group, or a combination thereof, R³ and R⁴ may be linked to each other to form a fused ring, and R⁵ and R⁶ may be linked to each other to form a fused ring.

In addition, the R¹ and R² may be each independently a substituted or unsubstituted C6 to C30 aryl group. Light emission in a visible region may be adjusted by controlling a pi conjugation length (π-conjugation length) of the R¹ and R².

Thereby, the compound may be usefully applied to an emission layer of an organic optoelectronic device.

More specifically, the R¹ and R² may be a fused substituted or unsubstituted C6 to C30 aryl group.

More specifically, the R¹ and R² may be each independently hydrogen, deuterium, a naphthyl group, a phenanthrenyl group, or an anthracenyl group but are not limited thereto.

Specific examples of the compound for an organic optoelectronic device are the following compounds, but are not limited thereto.

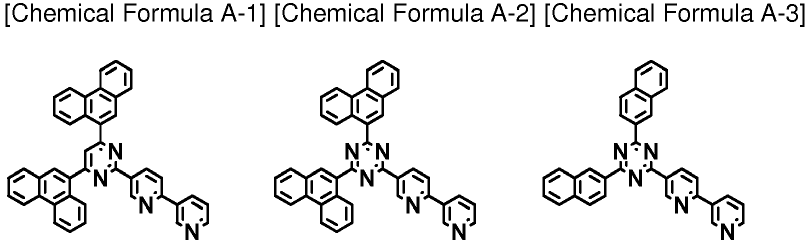

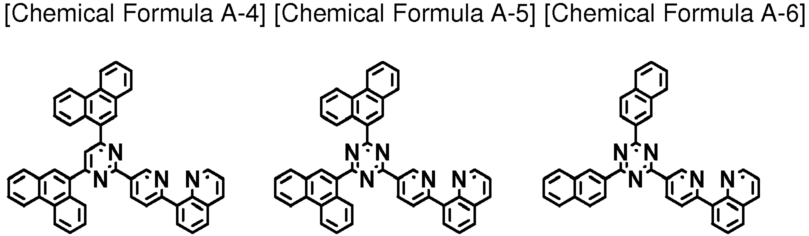

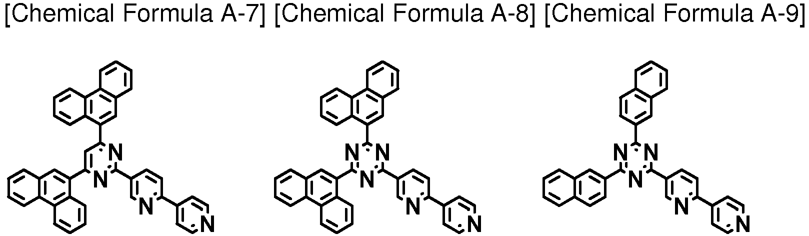

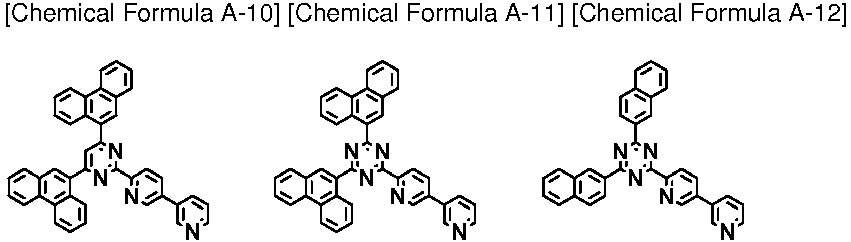

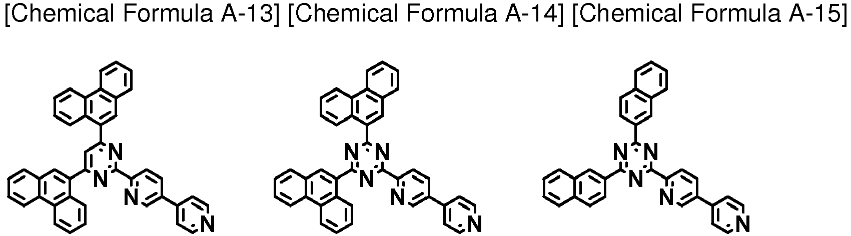

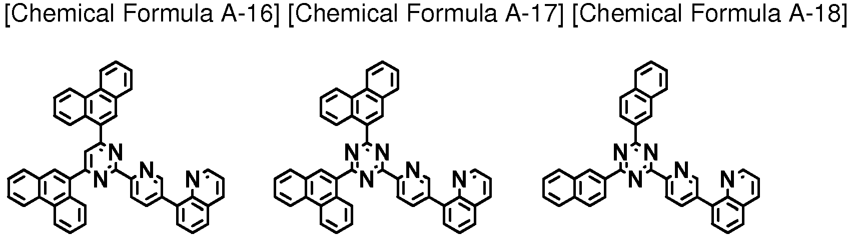

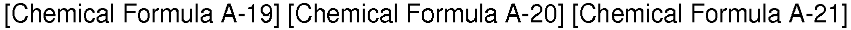

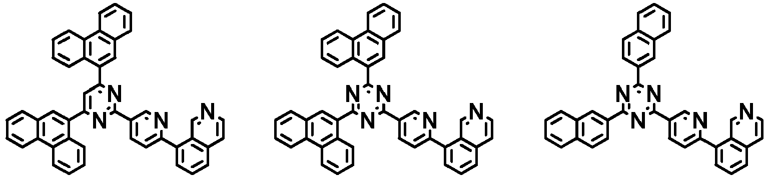

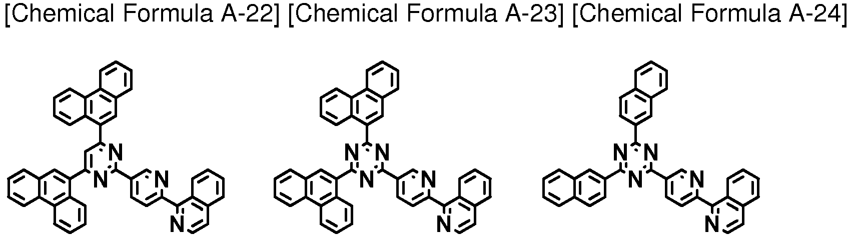

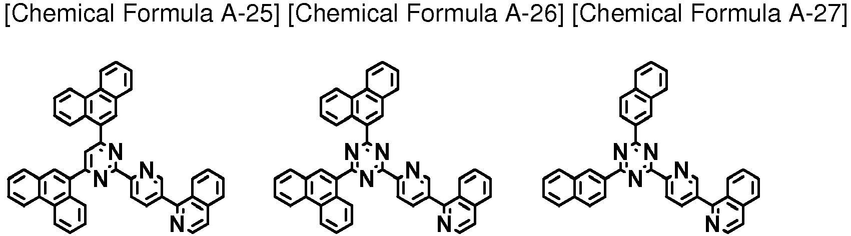

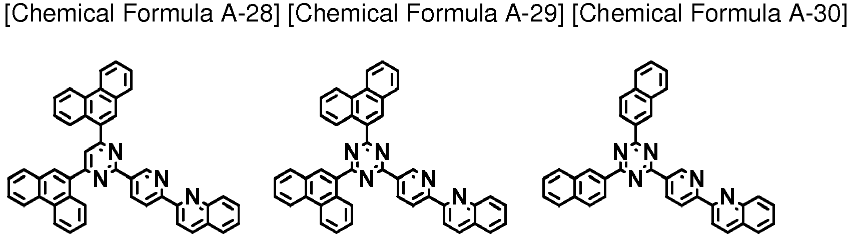

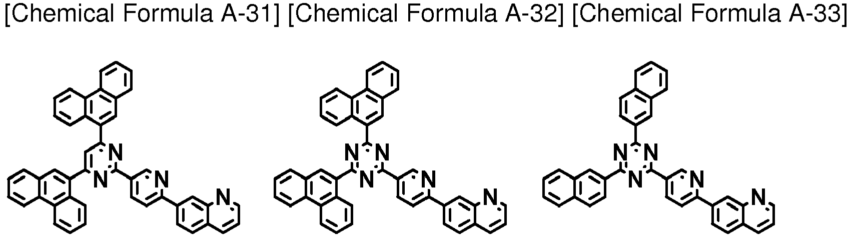

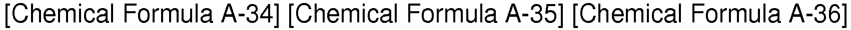

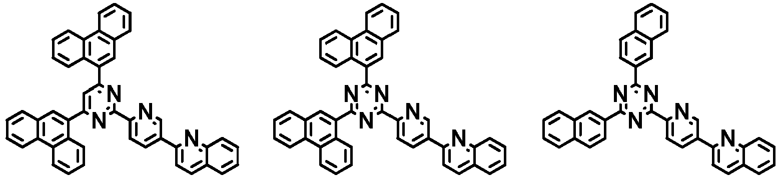

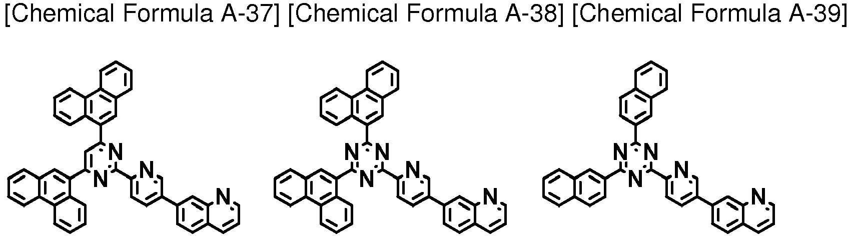

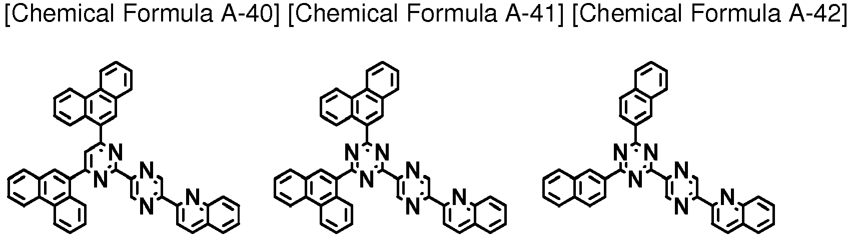

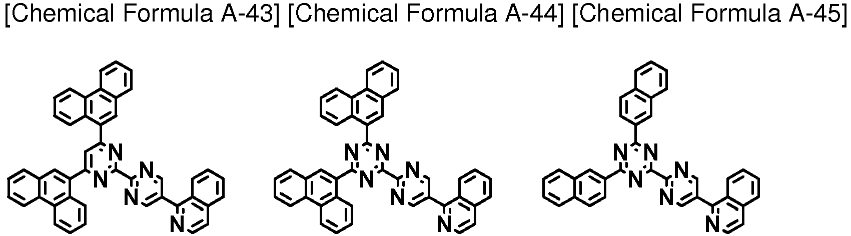

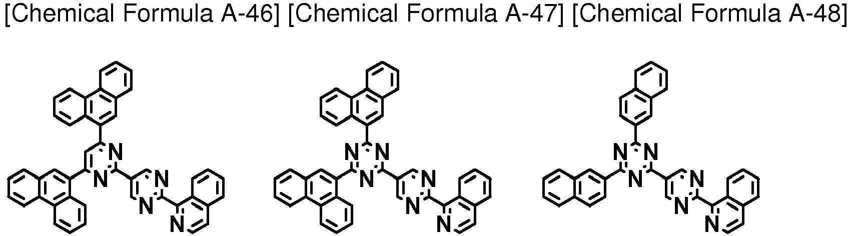

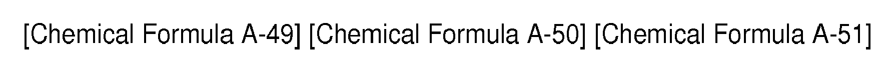

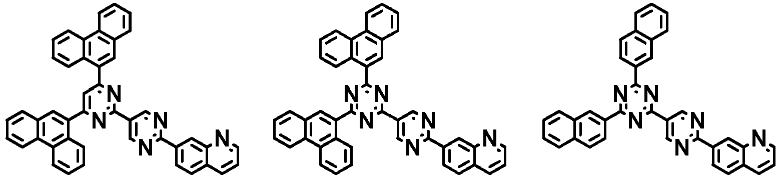

When the compound according to one embodiment of the present invention requires both electron characteristics and hole characteristics, introduction of a functional group having the electron characteristics thereinto has an effect on improving life-span and decreasing a driving voltage of an organic light-emitting device.

The compound for an organic optoelectronic device has a maximum light emitting wavelength in a range of about 320 to about 520 nm and a triplet excited energy (T1) ranging from greater than or equal to about 2.0 eV, and specifically, from about 2.0 to about 4.0 eV, and thus may well transport a host charge having high triplet excited energy to a dopant and increase luminous efficiency of the dopant, and is also freely adjusted regarding HOMO and LUMO energy levels and decreases a driving voltage, and accordingly may be usefully applied as a host material or a charge transport material.

In addition, the compound for an organic optoelectronic device has photoactive and electrical activities, and thus may be usefully applied for a nonlinear optic material, an electrode material, a discolored material, a light switch, a sensor, a module, a wave guide, an organic transistor, a laser, a light absorbent, a dielectric material, a separating membrane, and the like.

The compound for an organic optoelectronic device including the compounds has a glass transition temperature of greater than or equal to 90 °C and a thermal decomposition temperature of greater than or equal to 400 °C, indicating improved thermal stability. Thereby, it is possible to produce an organic optoelectronic device having high efficiency.

The compound for an organic optoelectronic device including the compounds may play a role of emitting light or injecting and/or transporting electrons, and may also act as a light emitting host with an appropriate dopant. In other words, the compound for an organic optoelectronic device may be used as a phosphorescent or fluorescent host material, a blue light emitting dopant material, or an electron transport material.

Since the compound for an organic optoelectronic device according to one embodiment is used for an organic thin layer, and it may improve the life-span characteristic, efficiency characteristic, electrochemical stability, and thermal stability of an organic optoelectronic device, and decrease the driving voltage.

Further, according to another embodiment, an organic optoelectronic device that includes the compound for an organic optoelectronic device is provided. The organic optoelectronic device may include an organic photoelectric device, an organic light-emitting device, an organic solar cell, an organic transistor, an organic photoconductor drum, an organic memory device, and the like. Particularly, the compound for an organic optoelectronic device according to one embodiment may be included in an electrode or an electrode buffer layer in an organic solar cell to improve the quantum efficiency, and it may be used as an electrode material for a gate, a source-drain electrode, or the like in the organic transistor.

Hereinafter, an organic light-emitting device is described.

According to another embodiment of the present invention, an organic light-emitting device includes an anode, a cathode, and at least one organic thin layer between the anode and the cathode, and at least one organic thin layer may include the compound for an organic optoelectronic device according to one embodiment of the present invention.

The organic thin layer that may include the compound for an organic optoelectronic device may include a layer selected from the group consisting of an emission layer, a hole transport layer (HTL), a hole injection layer (HIL), an electron transport layer (ETL), an electron injection layer (EIL), a hole blocking layer, and a combination thereof. The at least one layer includes the compound for an organic optoelectronic device according to one embodiment. Particularly, the compound for an organic optoelectronic device according to one embodiment may be included in a hole transport layer (HTL) or a hole injection layer (HIL). In addition, when the compound for an organic optoelectronic device is included in the emission layer, the compound for an organic optoelectronic device may be included as a phosphorescent or fluorescent host, and particularly, as a fluorescent blue dopant material.

FIGS. 1 and 2 are cross-sectional views showing organic light-emitting devices including the compound for an organic optoelectronic device according to one embodiment of the present invention.

Referring to FIGS. 1 and 2, organic light-emitting devices 100, 200, 300, 400, and 500 according to one embodiment include at least one organic thin layer 105 interposed between an anode 120 and a cathode 110.

The anode 120 includes an anode material having a large work function to help hole injection into an organic thin layer. The anode material includes: a metal such as nickel, platinum, vanadium, chromium, copper, zinc, and gold, or alloys thereof; a metal oxide such as zinc oxide, indium oxide, indium tin oxide (ITO), and indium zinc oxide (IZO); a combination of a metal and an oxide such as ZnO:Al and SnO₂:Sb; or a conductive polymer such as poly(3-methylthiophene), poly[3,4-(ethylene-1,2-dioxy)thiophene] (PEDT), polypyrrole, and polyaniline, but is not limited thereto. It is preferable to include a transparent electrode including indium tin oxide (ITO) as an anode.

The cathode 110 includes a cathode material having a small work function to help electron injection into an organic thin layer. The cathode material includes: a metal such as magnesium, calcium, sodium, potassium, titanium, indium, yttrium, lithium, gadolinium, aluminum, silver, tin, and lead, or alloys thereof; or a multi-layered material such as LiF/Al, Liq/Al, LiO₂/Al, LiF/Ca, LiF/Al, and BaF₂/Ca, but is not limited thereto. It is preferable to include a metal electrode including aluminum as a cathode.

First, referring to FIG. 1, the organic light-emitting device 100 includes an organic thin layer 105 including only an emission layer 130.

Referring to FIG. 2, a double-layered organic light-emitting device 200 includes an organic thin layer 105 including an emission layer 230 including an electron transport layer (ETL), and a hole transport layer (HTL) 140. As shown in FIG. 2, the organic thin layer 105 includes a double layer of the emission layer 230 and the hole transport layer (HTL) 140. The emission layer 130 also functions as an electron transport layer (ETL), and the hole transport layer (HTL) 140 layer has an improved binding property with a transparent electrode such as ITO or an improved hole transport capability. The organic thin layer 105 may further include an electron injection layer (EIL), an electron transport layer (ETL), an auxiliary electron transport layer (ETL), an auxiliary hole transport layer, a hole injection layer and a combination thereof even though they are not shown in FIGS. 1 or 2.

In FIGS. 1 and 2, at least one organic thin layer 105 selected from the emission layers 130 and 230, the hole transport layer (HTL) 140, even though being not shown, the electron injection layer (EIL), the electron transport layer (ETL), the auxiliary electron transport layer (ETL), the auxiliary hole transport layer (HTL), the hole injection layer (HIL), and a combination thereof may include the compound for an organic optoelectronic device. Herein, the compound for an organic optoelectronic device may be used in the electron transport layer (ETL) or an electron transport layer (ETL) including an electron injection layer (EIL), and when it is used in an electron transport layer (ETL), hole blocking layer (not shown) needs not to be formed separately to provide an organic light-emitting device having a simplified structure.

When the compound for an organic optoelectronic device is included in the emission layers 130 and 230, the compound for an organic optoelectronic device may be as a phosphorescent or fluorescent host.

The organic light-emitting device may be fabricated by: forming an anode on a substrate; forming an organic thin layer in accordance with a dry coating method such as evaporation, sputtering, plasma plating, and ion plating, or a wet coating method such as spin coating, dipping, and flow coating; and providing a cathode thereon.

Another embodiment of the present invention provides a display device including the organic light-emitting device according to the embodiment.

Hereinafter, the embodiments are illustrated in more detail with reference to examples. These examples, however, are not in any sense to be interpreted as limiting the scope of the invention.

### (Preparation of Compound for Organic optoelectronic device)

### Example 1: Synthesis of Compound of Chemical Formula A-1

A compound of the above Chemical Formula A-1 as specific examples of a compound for an organic opotoelectronic device of the present invention was synthesized through the following Reaction Scheme 1.

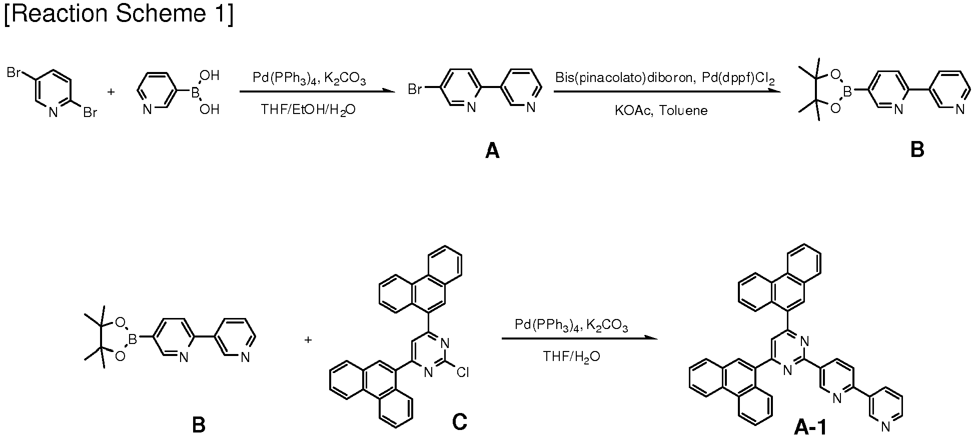

### First Step; Synthesis of Intermediate Product A

160.0 g (675 mmol) of 2,5-dibromopyridine, 83.02 g (675 mmol) of 3-pyridine boronic acid and 23.4 g (20.2 mmol) of tetrakis(triphenylphosphine)palladium [Pd(PPh₃)₄] were dissolved in 3.2 L of tetrahydrofuran (THF) and 1.6 L of ethanol as a solvent, a solution obtained by dissolving 186.68 g (1.35 mol) of potassium carbonate (K₂CO₃) in 1.6 L of water was added thereto, and the mixture was reacted at 90 °C for 18 hours. After checking if the reaction was complete by using TLC, the resultant was cooled down to room temperature and extracted. Then, normal hexane was added to the extract, the mixture was agitated, and a solid produced therein was filtered. The obtained solid was dried, obtaining 136.6 g of a compound intermediate A (a yield: 86 %).

### Second Step; Synthesis of Intermediate Product B

158.5 g (674 mmol) of the intermediate product A, 205.46 g (809 mmol) of bispinacolato diboron, 158.8 g (1.62 mol) of potassium acetate and 16.52 g (20.2 mmol) of 1,1'-bisdiphenylphosphinoferrocenedichloropalladium (II) [Pd(dppf)Cl₂] were reacted in 1.6 L of a toluene solvent at 110 °C for 14 hours. After checking if the reaction was complete by using TLC, the resultant was cooled down to room temperature and then, extracted. Then, an organic layer obtained therefrom was treated with magnesium sulfate (MgSO₄) to remove moisture therefrom and purified through column chromatography (Hexanes:EA = 1:1 v/v). The obtained compound was recrystallized with methanol, obtaining 77 g of a compound intermediate B (a yield: 40.5 %).

### Third Step: Synthesis of Compound A-1 represented by Chemical Formula 1

16.6 g (58.9 mmol) of the intermediate product B, 25 g (53.5 mmol) of the compound C and 1.85 g (1.61 mmol) of tetrakis(triphenylphosphine)palladium [Pd(PPh₃)₄] were dissolved in 500 ml of a tetrahydrofuran (THF) solvent, a solution obtained by dissolving 16.28 g (118 mmol) of potassium carbonate (K₂CO₃) in 250 ml of water was added thereto, and the mixture was reacted at 90 °C for 12 hours. After checking if the reaction was complete by using TLC, the resultant was cooled down. Then, the solution was filtered and washed with a large amount of methanol and water. The obtained solid was dissolved in dichlorobenzene and recrystallized with methanol, obtaining 22.1 g of a compound of Chemical Formula A-1 (a yield: 70.4 %). (a calculation value: 586.68, a measurement value: MS[M+1] 587)

### Example 2: Synthesis of Compound of Chemical Formula A-2

A compound of the above Chemical Formula A-2 as specific examples of a compound for an organic optoelectronic device according to the present invention was synthesized through the following Reaction Scheme 2.

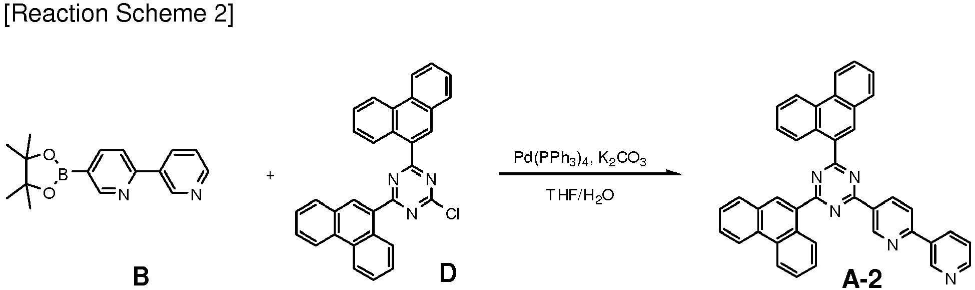

### First step: Synthesis of Compound of Chemical Formula A-2

16.58 g (58.8 mmol) of the intermediate product B, 25 g (53.4 mmol) of the compound D and 1.85 g (1.6 mmol) of tetrakis(triphenylphosphine)palladium [Pd P(Ph₃)₄] were dissolved in 500 ml of a tetrahydrofuran (THF) solvent, a solution obtained by dissolving 16.24 g (118 mmol) of potassium carbonate (K₂CO₃) in 250 ml of water was added thereto, and the mixture was reacted at 90 °C for 12 hours. After checking if the reaction was complete by using TLC, the resultant was cooled down. Then, the solution was filtered and then, washed with a large amount of methanol and water. The obtained solid was dissolved in dichlorobenzene and then, recrystallized with methanol, obtaining 29.6 g of a compound of Chemical Formula A-2 (a yield: 70.7 %). (a calculation value: 587.67, a measurement value: MS[M+1] 588)

### Example 3: Synthesis of Compound of Chemical Formula A-6

A compound of the above Chemical Formula A-6 as specific examples of a compound for an organic optoelectronic device according to the present invention was synthesized through the following Reaction Scheme 3.

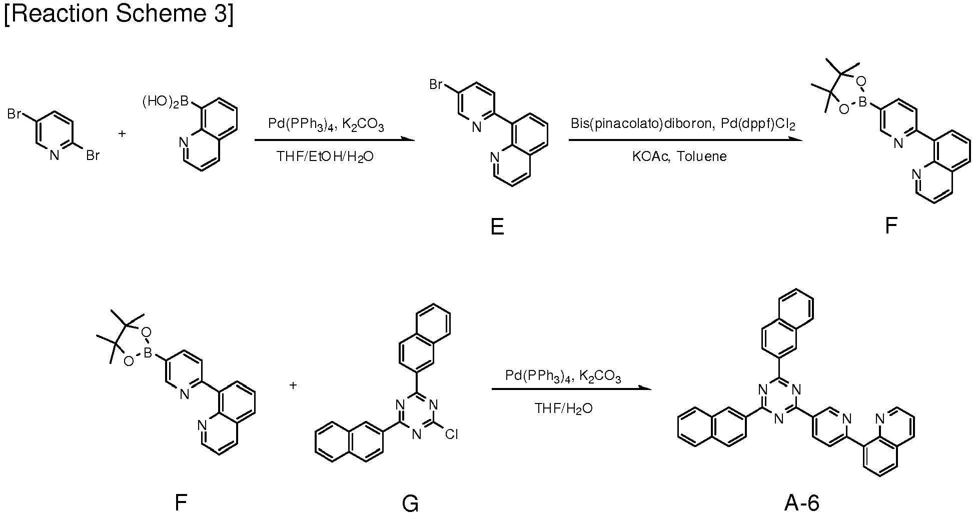

### First Step; Synthesis of Intermediate Product E

20.0 g (84.4 mmol) of 2,5-dibromopyridine, 16.06 g (92.9 mmol) of 8-quinolineboronic acid and 2.93 g (2.53 mmol) of tetrakis(triphenylphosphine)palladium [Pd(PPh₃)₄] were dissolved in 400 ml of tetrahydrofuran (THF) and 140 ml of ethanol as a solvent, a solution obtained by dissolving 22.34 g (168.89 mmol) of potassium carbonate (K₂CO₃) in 140 ml of water was added thereto, and the mixture was reacted at 90 °C for 12 hours. After checking if the reaction was complete by using TLC, the resultant was cooled down to room temperature and then, extracted. Then, an organic layer obtained therefrom was treated with magnesium sulfate (MgSO₄) to remove moisture and then, purified through column chromatography (Hexanes:EA=4:1 v/v). The obtained compound was recrystallized with normal hexane, obtaining 15.44 g of a compound intermediate E (a yield: 64 %).

### Second Step; Synthesis of Intermediate Product F

15.0 g (58.1 mmol) of the intermediate product E, 17.71 g (69.7 mmol) of bispinacolato diboron, 17.11 g (174.3 mmol) of potassium acetate and 1.42 g (1.74 mmol) of 1,1'-bisdiphenylphosphinoferrocenedichloropalladium (II) [Pd(dppf)Cl₂] were reacted in 300 ml of a toluene solvent at 110° C for 4 hours. After checking if the reaction was complete by using TLC, the resultant was cooled down. Then, a solvent therein was removed under a reduced pressure, and a product obtained therefrom was rinsed with water and methanol. The residue was recrystallized with toluene, and a solid extracted therefrom was separated through a filter and then, rinsed with toluene and dried, obtaining 10 g of a solid intermediate product F (a yield: 52 %).

### Third Step: Synthesis of Compound of Chemical Formula A-6

10.0 g (30.1 mmol) of the intermediate product F, 9.96 g (27.1 mmol) of the compound G and 0.94 g (0.81 mmol) of tetrakis(triphenylphosphine)palladium [Pd(PPh₃)₄] were dissolved in 200 ml of a tetrahydrofuran (THF) solvent, a solution obtained by dissolving 7.5 g (54.2 mmol) of potassium carbonate (K₂CO₃) in 100 ml of water, and the mixture was reacted at 90 °C for 12 hours. After checking if the reaction was complete by using TLC, the resultant was cooled down. Then, the solution was filtered and washed with a large amount of methanol and water. The obtained solid was dissolved in dichlorobenzene and then, recrystallized with methanol, obtaining 7.5 g of a compound of Chemical Formula A-6 (a yield: 52 %). (a calculation value: 537.61, a measurement value: MS[M+1] 538)

### (Manufacture of Organic Light-Emitting Device)

### Example 4

As for an anode, a 1000 Å-thick ITO was used, and as for a cathode, a 1000 Å-thick aluminum (Al) was used.

Specifically, the anode was manufactured by cutting an ITO glass substrate having sheet resistance of 15 Ω/cm² into a size of 50 mm x 50 mm x 0.7 mm and washing it with a ultrasonic wave in acetone, isopropyl alcohol, and pure water respectively for 15 minutes and then, with UV ozone for 30 minutes.

On the glass substrate, a 65 nm-thick hole injection layer (HIL) was formed by depositing N1,N1'-(biphenyl-4,4'-diyl)bis(N1-(naphthalen-2-yl)-N4,N4-diphenylbenzene-1,4-diamine), and subsequently a 40 nm-thick hole transport layer (HTL) was formed by depositing N,N'-di(1-naphthyl)-N,N'-diphenylbenzidine.

A 25 nm-thick emission layer was formed by depositing 4 % of N,N,N',N'-tetrakis (3,4-dimethylphenyl)chrysene-6,12-diamine and 96 % of 9-(3-(naphthalen-1-yl)phenyl)-10-(naphthalen-2-yl)anthracene.

Subsequently, a 30 nm-thick electron transport layer (ETL) was formed by depositing the compound prepared in the Example 1.

On the electron transport layer, for an electron injection layer (EIL), a Liq/Al electrode was formed by depositing 0.5 nm-thick Liq and 100 nm-thick Al.

### Example 5

An organic light-emitting device was manufactured according to the same method as Example 4 except using the compound prepared in Example 3 for an electron transport layer (ETL), instead of the compound prepared in Example 1.

### Comparative Example 1

An organic light-emitting device was manufactured according to the same method as Example 4 except using the compound represented by Chemical Formula R-1 for an electron transport layer (ETL), instead of the compound prepared in Example 1.

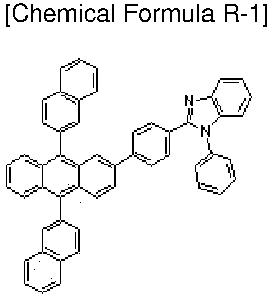

### (Performance Measurement of Organic Light-Emitting Device)

### Experimental Example

Current density and luminance changes depending on a voltage and luminous efficiency of each organic light-emitting device according to Examples 4 and 5 and Comparative Example 1 were measured. The measurements were specifically performed in the following method, and the results were provided in the following Table 1.

### (1) Measurement of Current Density Change Depending on Voltage Change

The obtained organic light-emitting devices were measured for current value flowing in the unit device while increasing the voltage from 0 V to 10 V using a current-voltage meter (Keithley 2400), the measured current value was divided by area to provide the results.

### (2) Measurement of Luminance Change Depending on Voltage Change

Luminance was measured by using a luminance meter (Minolta Cs-1000A), while the voltage of the organic light-emitting devices was increased from 0 V to 10 V.

### (3) Measurement of Luminous Efficiency

Current efficiency (cd/A) and power efficiency (Im/W) at the same luminance (1000 cd/m²) were calculated by using the luminance, current density, and voltages (V) from the items (1) and (2).

**[Table 1]**

| | Luminance 500 cd/m² | | | | |
|---|---|---|---|---|---|
| | Driving voltage (V) | Luminous efficiency (cd/A) | Power efficiency | CIE color coordinate | |
| | | | (Im/W) | x | y |
| Example 4 | 4.7 | 3.9 | 2.6 | 0.14 | 0.05 |
| Example 5 | 5.0 | 3.6 | 2.3 | 0.14 | 0.05 |
| **Comparative Example 1** | **5.1** | **3.7** | **2.3** | **0.14** | **0.05** |

As shown in Table 1, the organic light-emitting devices according to Examples 4 and 5 showed a lower driving voltage, and excellent luminous efficiency, and/or power efficiency compared with the organic light-emitting device according to Comparative Example 1.

While this invention has been described in connection with what is presently considered to be practical exemplary embodiments, it is to be understood that the invention is not limited to the disclosed embodiments, but, on the contrary, is intended to cover various modifications and equivalent arrangements included within the spirit and scope of the appended claims. Therefore, the aforementioned embodiments should be understood to be exemplary but not limiting the present invention in any way.

### <Description of Symbols>

| | |
|---|---|
| 100: organic light-emitting device | 110: cathode |
| 120: anode | 105: organic thin layer |
| 130: emission layer | 140: hole transport layer (HTL) |
| 230: emission layer + electron transport layer (ETL) | |

## Claims

1. A compound represented by the following Chemical Formula 1 for an organic optoelectronic device: 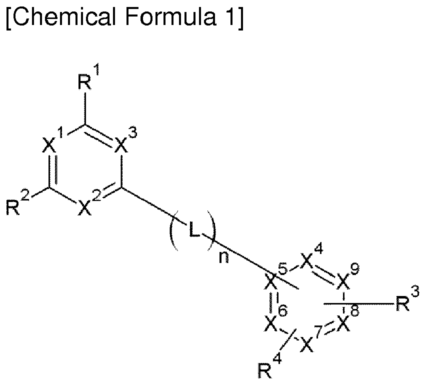 wherein, in Chemical Formula 1,
X¹ to X⁹ are each independently CR' or N, at least two of the X¹ to X³ is N, at least one of the X⁴ to X⁹ is N,
R¹ to R⁴ and R' are each independently hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 alkylthiol group, a substituted or unsubstituted C6 to C20 arylthiol group, a substituted or unsubstituted C1 to C20 heterocyclothiol group, a substituted or unsubstituted C1 to C20 ureide group, a substituted or unsubstituted C3 to C40 silyl group, or a combination thereof,
R³ and R⁴ are optionally linked to each other to form a fused ring,
L is a substituted or unsubstituted C2 to C30 heteroarylene group, and
n is an integer ranging from 1 to 3.

2. The compound for an organic optoelectronic device of claim 1, wherein the L is a substituted or unsubstituted C2 to C30 heteroarylene group including one or two nitrogen.

3. The compound for an organic optoelectronic device of claim 1, wherein the R¹ and R² are each independently a substituted or unsubstituted C6 to C30 aryl group.

4. The compound for an organic optoelectronic device of claim 1, wherein the compound for an organic optoelectronic device is represented by the following Chemical Formula 2: 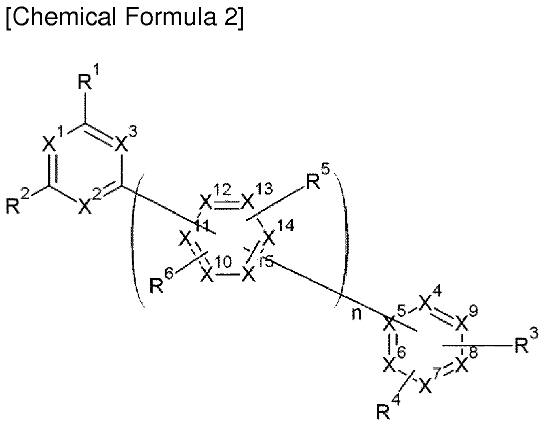 wherein, in Chemical Formula 2,
X¹ to X¹⁵ are each independently CR' or N, at least two of the X¹ to X³ are N, at least one of the X⁴ to X⁹ is N, at least one of the X¹⁰ to X¹⁵ is N,
R¹ to R⁶ and R' are each independently hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 alkylthiol group, a substituted or unsubstituted C6 to C20 arylthiol group, a substituted or unsubstituted C1 to C20 heterocyclothiol group, a substituted or unsubstituted C1 to C20 ureide group, a substituted or unsubstituted C3 to C40 silyl group, or a combination thereof,
R³ and R⁴ are optionally linked to each other to form a fused ring,
R⁵ and R⁶ are optionally linked to each other to form a fused ring,
n is an integer ranging from 1 to 3.

5. The compound for an organic optoelectronic device of claim 4, wherein one of the X¹⁰ to X¹⁵ is N.

6. The compound for an organic optoelectronic device of claim 5, wherein the n is 1.

7. The compound for an organic optoelectronic device of claim 5, wherein the R¹ and R² are each independently hydrogen, deuterium, a naphthyl group, a phenanthrenyl group, or an anthracenyl group, and
the R⁴, R⁷ and R⁸ are hydrogen.

8. The compound for an organic optoelectronic device of claim 1, wherein the compound for an organic optoelectronic device is represented by the following Chemical Formula 3: 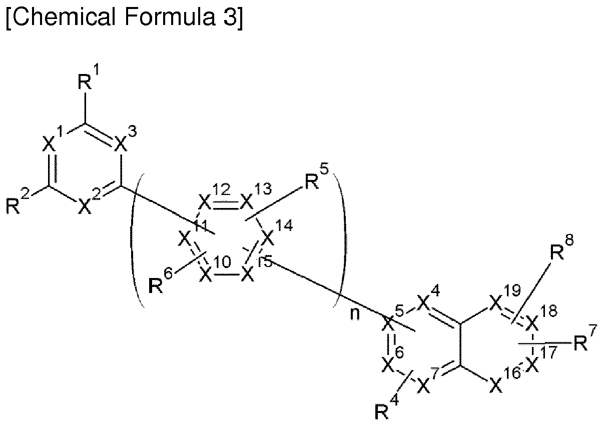 wherein, in Chemical Formula 3,
X¹ to X⁷, X¹⁰ to X¹⁵ and X¹⁶ to X¹⁹ are each independently CR' or N, at least two of the X¹ to X³ are N, at least one of the X⁴ to X⁷ and X¹⁶ to X¹⁹ is N, at least one of the X¹⁰ to X¹⁵ is N,
R¹, R⁴ to R⁸ and R' are each independently hydrogen, deuterium, a halogen, a cyano group, a hydroxyl group, an amino group, a substituted or unsubstituted C1 to C20 amine group, a nitro group, a carboxyl group, a ferrocenyl group, a substituted or unsubstituted C1 to C20 alkyl group, a substituted or unsubstituted C6 to C30 aryl group, a substituted or unsubstituted C2 to C30 heteroaryl group, a substituted or unsubstituted C1 to C20 alkoxy group, a substituted or unsubstituted C6 to C20 aryloxy group, a substituted or unsubstituted C3 to C40 silyloxy group, a substituted or unsubstituted C1 to C20 acyl group, a substituted or unsubstituted C2 to C20 alkoxycarbonyl group, a substituted or unsubstituted C2 to C20 acyloxy group, a substituted or unsubstituted C2 to C20 acylamino group, a substituted or unsubstituted C2 to C20 alkoxycarbonylamino group, a substituted or unsubstituted C7 to C20 aryloxycarbonylamino group, a substituted or unsubstituted C1 to C20 sulfamoylamino group, a substituted or unsubstituted C1 to C20 sulfonyl group, a substituted or unsubstituted C1 to C20 alkylthiol group, a substituted or unsubstituted C6 to C20 arylthiol group, a substituted or unsubstituted C1 to C20 heterocyclothiol group, a substituted or unsubstituted C1 to C20 ureide group, a substituted or unsubstituted C3 to C40 silyl group, or a combination thereof,
R⁷ and R⁸ are optionally linked to each other to form a fused ring,
R⁵ and R⁶ are optionally linked to each other to form a fused ring, and
n is an integer ranging from 1 to 3.

9. The compound for an organic optoelectronic device of claim 8, wherein the R¹ and R² are each independently hydrogen, deuterium, a naphthyl group, a phenanthrenyl group, or an anthracenyl group, and
the R⁴, R⁷ and R⁸ are hydrogen.

10. The compound for an organic optoelectronic device of claim 8, wherein at least one of the X⁴ to X⁷ is N, and the X¹⁶ to X¹⁹ is CR'.

11. The compound for an organic optoelectronic device of claim 8, wherein the X⁴ to X⁷ are CR', and at least one of the X¹⁶ to X¹⁹ is N.

12. The compound for an organic optoelectronic device of claim 1, wherein the compound for an organic optoelectronic device has triplet exciton energy (T1) of greater than or equal to 2.0eV.

13. The compound for an organic optoelectronic device of claim 1, wherein the organic optoelectronic device is selected from an organic photoelectric device, an organic light-emitting device, an organic solar cell, an organic transistor, an organic photoconductor drum, and an organic memory device.

14. An organic light-emitting device comprising
an anode, a cathode, and at least one organic thin layer between the anode and the cathode,
wherein at least one of the organic thin layer comprises the compound for an organic optoelectronic device of claim 1.

15. The organic light-emitting device of claim 14, wherein the organic thin layer is selected from an emission layer, a hole transport layer (HTL), a hole injection layer (HIL), an electron transport layer (ETL), an electron injection layer (EIL), a hole blocking layer and a combination thereof.

16. The organic light-emitting device of claim 15, wherein the compound for an organic optoelectronic device is included in an electron transport layer (ETL).

17. A display device comprising the organic light-emitting device of claim 14.
